Europäisches Patentamt

European Patent Office

Office européen des brevets

(19)

(11) Veröffentlichungsnummer: **0 268 850 B1**

# (12) EUROPÄISCHE PATENTSCHRIFT

(45) Veröffentlichungstag der Patentschrift: **11.12.91**

(51) Int. Cl.⁵: **A61N 1/08**

(21) Anmeldenummer: **87115685.7**

(22) Anmeldetag: **26.10.87**

(54) **Reizstromgerät.**

(30) Priorität: **07.11.86 DE 3638014**

(43) Veröffentlichungstag der Anmeldung:
**01.06.88 Patentblatt 88/22**

(45) Bekanntmachung des Hinweises auf die
Patenterteilung:
**11.12.91 Patentblatt 91/50**

(84) Benannte Vertragsstaaten:
**AT BE CH DE FR LI NL**

(56) Entgegenhaltungen:
**US-A- 4 177 819**
**US-A- 4 327 326**

(73) Patentinhaber: **SIEMENS AKTIENGESELL-
SCHAFT**
**Wittelsbacherplatz 2**
**W-8000 München 2(DE)**

(72) Erfinder: **Helmreich, Klaus**
**Rhönstrasse 36**
**W-8520 Erlangen(DE)**
Erfinder: **Herzog, Ludwig**
**Drei-Thorn-Strasse 3**
**W-6948 Waldmichelbach(DE)**
Erfinder: **Knapp, Volker**
**Pestalozzistrasse 19**
**W-6948 Waldmichelbach(DE)**

EP 0 268 850 B1

## Beschreibung

Die Erfindung bezieht sich auf ein Reizstromgerät gemäß Oberbegriff des Patentanspruchs 1.

Bei Geräten der genannten Art besteht Gefahr, daß die Intensität zu hoch eingestellt wird (mit für den Patienten unter Umständen schmerzhaften Folgen), nämlich dann, wenn eine Intensitätsänderung am Intensitätseinstellglied vorgenommen wird zu einer Zeit in der der Schwellzyklus gerade eine Pause (Nullinie) durchläuft. In US-A-4 177 819 ist ein Reizstromgerät mit einer Sicherheitsschaltung beschrieben, die das Anlegen eines Reizstromes verhindert, wenn die Intensität des Reizstromes bei Beginn der Behandlung nicht auf null gestellt ist.

Aufgabe vorliegender Erfindung ist es, ein Reizstromgerät aufzubauen, bei dem die Intensität nicht unkontrolliert gesteigert werden kann.

Die Aufgabe wird erfindungsgemäß durch die kennzeichnenden Merkmale des Patentanspruchs 1 gelöst.

Gemäß der Erfindung beginnt der Schwellzyklus von vorn, sobald das Intensitätseinstellglied betätigt wird. Die Intensitätseinstellung kann jetzt nicht mehr in eine Schwellpause fallen. Sie kann jetzt vielmehr im ansteigenden Ast der Schwellung kontrolliert werden.

Weitere Vorteile und Einzelheiten der Erfinung ergeben sich aus der nachfolgenden Beschreibung eines Ausführungsbeispiels anhand der Zeichnung und in Verbindung mit den Unteransprüchen.

Es zeigen:

Fig. 1      das erfindungsgemäße Reizstromgerät im Prinzipschaltbild und

Fig. 2 bis 4      ein Impulsdiagramm einer Schwellung in Abhängigkeit von einer Intensitätsänderung.

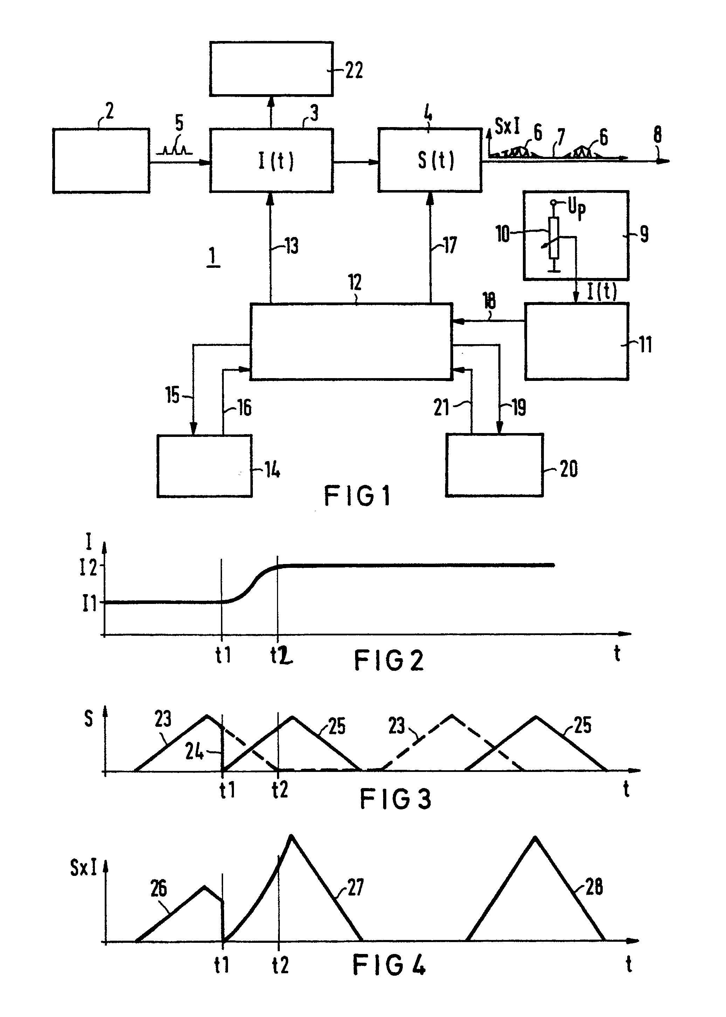

In der Fig. 1 umfaßt das Reizstromgerät 1 unter anderem einen Reizimpulserzeuger 2, dem eine Intensitätseinstelleinrichtung 3 und eine Schwelleinrichtung 4 zum zyklischen An- bzw. Wiederabschwellen der Amplitude der Reizimpulse 5 des Reizimpulserzeugers 2 nachgeschaltet sind. Die Intensitätseinstelleinrichtung 3 und die Schwelleinrichtung 4 arbeiten dabei in der Weise zusammen, daß sich ihre Signale multiplikativ zum zyklisch auf- und abschwellenden Reizsignal 6 überlagern. Das Reizsignal 6 mit Pausen 7 wird (wie durch einen Pfeil 8 angedeutet ist) dem Reizausgang des Reizstromgerätes 1 mit den dort angeschlossenen Reizstromelektroden (nicht dargestellt) zugeleitet.

Zur Einstellung der Intensität ist ein Intensitätseinstellglied 9 vorgesehen (Drehpotentiometer 10, das an einer Spannung $U_p$ liegt).

Dem Intensitätseinstellglied 9 ist ein Analog-Digital-Wandler 11 nachgeschaltet, der den Istwert der Einstellung am Intensitätseinstellglied periodisch rasch, z.B. 20 mal pro Sekunde, abtastet.

Ein Mikroprozessor 12, der über eine Leitung 13 die Intensitätseinstelleinrichtung 3 in Abhängigkeit vom eingestellten Intensitätswert steuert und der auch mit der Schwelleinrichtung 4 zur Einstellung bestimmter, in einem EPROM 14 (mit Dialogleitungen 15, 16) gespeicherten Schwellformen über eine Steuerleitung 17 in Verbindung steht, ruft über eine Leitung 18 die vom Analog-Digital-Wandler 11 gelieferten digitalen Istwerte der Intensitätseinstellung ab. Jeder Istwert wird dann vom Mikroprozessor 12 über eine Leitung 19 in ein RAM 20 eingespeichert. Gleichzeitig ruft der Mikroprozessor 12 über die Leitung 21 den unmittelbar zuvor abgespeicherten Einstellwert aus dem RAM 20 ab und vergleicht ihn mit dem Istwert.

Bei einer Abweichung der beiden Werte (die Intensitätseinstellung am Intensitätseinstellglied 9 wurde oder wird noch geändert) erzeugt der Mikroprozessor 12 erfindungsgemäß auf der Leitung 17 ein Signal, das die Schwelleinrichtung 4 in dem Sinne steuert, daß der momentan ablaufende Schwellzyklus auf den Ausgangspunkt (Null) zurückgesetzt und anschließend erneut gestartet wird. Die über den Mikroprozessor 12 und die Leitung 13 der Intensitätseinstelleinrichtung 3 mitgeteilte Intensitätsänderung erfolgt jetzt im aufsteigenden Ast einer Schwellung und kann direkt an einer Intensitätsanzeige 22 beobachtet werden. Eine zufällige Einstellung während einer Pause, die zu überhöhten Werten führen könnte, ist unter normalen Umständen nicht mehr möglich.

Die zuvor beschriebene Schwellsteuerung wird im Impulsdiagramm der Fig. 2 bis 4 verdeutlicht.

Fig. 2 zeigt das Ausgangssignal I(t) des Intensitätseinstellgliedes 9. Bis zum Zeitpunkt t1 ist der Einstellwert konstant I1. Zum Zeitpunkt t1 wird der Einstellwert erhöht. Die Einstellung I2 wird zum Zeitpunkt t2 erreicht und bleibt anschließend konstant.

Fig. 3 zeigt den Zyklusablauf S(t) in der Schwelleinrichtung 4. Zum Zeitpunkt t1 ist das Schwellsignal 23 im absteigenden Ast. Bei unverändertem Intensitätseinstellwert würde das Schwellsignal 23 dem gestrichelten Verlauf folgen. Tatsächlich bewirkt jedoch der Beginn der Intensitätswerterhöhung zum Zeitpunkt t1 den zuvor beschriebenen Sprung des Signales 23 zu Null, was in der Fig. 3 durch die senkrechte Linie 24 angedeutet ist. Anschließend wird ein neuer Schwellzyklus 25 gestartet.

Das multiplikativ überlagerte Ergebnis I(t) × S(t) am Ausgang der (multiplizierenden) Schwelleinrichtung 4 ist in Fig. 4 dargestellt. Der Signalanteil 26 bis t1 gehört noch zum vorausgegangenen Zyklus. Die Signalanteile 27, 28 mit entsprechend höherer Intensität stammen aus dem neuen Zyklus.

## Patentansprüche

1. Reizstromgerät, insbesondere für Reizstromtherapie an einem Patienten, mit einem Reizimpulserzeuger (2), einer Intensitätseinstelleinrichtung (3) mit Intensitätseinstellglied (9) und einer Reizimpuls-Schwelleinrichtung (4) zum An- bzw. Wiederabschwellen der Amplituden der Reizimpulse, **dadurch gekennzeichnet,** daß dem Intensitätseinstellglied (9) eine Abtasteinrichtung (11) zum Abtasten der eingestellten Intensität sowie ein Speicher (20) zur Speicherung des abgetasteten Intensitätseinstellwertes und eine Vergleichsvorrichtung (12) zum Vergleich neuer Intensitätseinstellwerte mit vorausgegangenen gespeicherten Intensitätseinstellwerten zugeordnet sind, wobei die Vergleichsvorrichtung (12) die Schwelleinrichtung (4) steuert in dem Sinne, daß diese auf den Ausgangspunkt eines Schwellzyklus zurückgesetzt wird, sobald die Vergleichsvorrichtung (12) eine Abweichung zwischen einem neuen (I2) und einem vorausgegangenen (I1) gespeicherten Intensitätseinstellwert festgestellt hat und daß sie anschließend einen neuen Schwellzyklus startet.

2. Reizstromgerät nach Anspruch 1, **dadurch gekennzeichnet,** daß die Intensitätseinstelleinrichtung (3) und die Schwelleinrichtung (4) zusammengeschaltet sind in dem Sinne, daß ihre Signale sich multiplikativ überlagern.

3. Reizstromgerät nach Anspruch 1 oder 2, **dadurch gekennzeichnet,** daß die Abtasteinrichtung (11) das Intensitätseinstellglied (9) ca. 20 mal pro Sekunde abtastet.

4. Reizstromgerät nach einem der Ansprüche 1 bis 3, **dadurch gekennzeichnet,** daß die Vergleichsvorrichtung (12) Bestandteil eines Mikroprozessors ist.

5. Reizstromgerät nach Anspruch 4, **dadurch gekennzeichnet,** daß die Abtasteinrichtung (11) ein mit dem Mikroprozessor (12) verbundener Analog-Digital-Wandler ist.

6. Reizstromgerät nach Anspruch 4 oder 5, **dadurch gekennzeichnet,** daß der Speicher (20) ein mit dem Mikroprozessor (12) im Dialog stehendes RAM ist.

## Claims

1. Stimulating current unit, in particular for stimulating current therapy on a patient, having a stimulating pulse generator (2), an intensity-adjusting device (3) with intensity-adjusting element (9) and a stimulating pulse-swelling device (4) for increasing, or decreasing again, the amplitudes of the stimulating pulses, characterised in that associated with the intensity-adjusting element (9) there is a scanning device (11) for scanning the adjusted intensity and also a memory (20) for storing the scanned adjusted intensity value and a comparison arrangement (12) for comparing new adjusted intensity values with previous stored adjusted intensity values, the comparison arrangement (12) controlling the swelling device (4) in the sense that the latter is reset to the starting point of a swelling cycle as soon as the comparison arrangement (12) has determined a deviation between a new (I2) and a previous (I1) stored adjusted intensity value and in that it subsequently starts a new swelling cycle.

2. Stimulating current unit according to claim 1, characterised in that the intensity-adjusting device (3) and the swelling device (4) are interconnected in the sense that their signals are superimposed upon each other in a multiplicative manner.

3. Stimulating current unit according to claim 1 or 2, characterised in that the scanning device (11) scans the intensity-adjusting element (9) approximately 20 times per second.

4. Stimulating current unit according to one of the claims 1 to 3, characterised in that the comparison arrangement (12) is part of a microprocessor.

5. Stimulating current unit according to claim 4, characterised in that the scanning device (11) is an analog-digital converter connected with the microprocessor (12).

6. Stimulating current unit according to claim 4 or 5, characterised in that the memory (20) is a RAM in interactive communication with the microprocessor (12).

## Revendications

1. Stimulateur, notamment pour la thérapie utilisant un courant de stimulation appliqué à un patient, et comportant un générateur d'impulsions de stimulation (2), un dispositif (3) de réglage de l'intensité comportant un organe (9) de réglage de l'intensité et un dispositif (4) de réglage du seuil des impulsions de stimulation, qui sert à augmenter ou réduire des seuils des amplitudes des impulsions de stimulation, caractérisé par le fait qu'à l'organe (9) de réglage de l'intensité sont associés un dispositif d'exploration (11) servant à explorer l'intensité réglée, ainsi qu'une mémoire (20) servant à mémoriser la valeur réglée explorée de l'intensité, et un dispositif comparateur (12) servant à comparer de nouvelles valeurs de réglage de l'intensité à des valeurs précédentes mémorisées de réglage de l'intensité, le dispositif comparateur (12) commandant le dispositif d'excitation (4) de manière à ramener ce dispositif au point de départ d'un cycle d'excitation, dès que le dispositif comparateur (12) a établi un écart entre une nouvelle valeur (I2) de réglage de l'intensité et une valeur précédente (I1) mémorisée de réglage de l'intensité et qu'il déclenche ensuite un niveau cycle d'excitation.

2. Stimulateur suivant la revendication 1, caractérisé par le fait que le dispositif (3) de réglage de l'intensité et le dispositif d'excitation (4) sont interconnectés de telle sorte que leurs signaux sont réunis de façon multiplicative.

3. Stimulateur suivant la revendication 1 ou 2, caractérisé par le fait que le dispositif d'exploration (11) explore l'organe (9) de réglage de l'intensité, environ 20 fois par seconde.

4. Stimulateur suivant l'une des revendications 1 à 3, caractérisé par le fait que le dispositif comparateur (12) fait partie d'un microprocesseur.

5. Stimulateur suivant la revendication 4, caractérisé par le fait que le dispositif d'exploration (11) est un convertisseur analogique/numérique raccordé au microprocesseur (12).

6. Stimulateur suivant la revendication 4 ou 5, caractérisé par le fait que la mémoire (20) est une mémoire RAM dialoguant avec le microprocesseur (12).

FIG 1

FIG 2

FIG 3

FIG 4